# EUROPEAN PATENT SPECIFICATION

(11) **EP 1 716 210 B1**
(45) Date of publication and mention of the grant of the patent: **05.10.2016**
(21) Application number: 05715277.9
(22) Date of filing: 08.02.2005
(51) Int. Cl.: C09B 61/00

(54) **COMPOSITIONS CONTAINING CIS-ISOMERS OF CAROTENOIDS AND CORRESPONDING METHOD**
ZUSAMMENSETZUNGEN MIT CIS-ISOMEREN VON CAROTINOIDEN UND ENTSPRECHENDES VERFAHREN
COMPOSITIONS CONTENANT DES ISOMÈRES CIS DE CAROTÉNOIDES ET PROCÉDÉ

(30) Priority: 10.02.2004 EP 04002853; 06.07.2004 EP 04015865
(43) Date of publication of application: 02.11.2006
(73) Proprietor: Nestec S.A., 1800 Vevey (CH)
(72) Inventor: BORTLIK, Karlheinz, CH-1510 Syens (CH); Richelle, Myriam, CH-1073 Savigny (CH); LAMBELET, Pierre, CH-1806 Saint-Légier (CH); SAUCY, Francoise, CH-1807 Blonay (CH)
(74) Representative: Rosolen-Delarue, Katell
(86) International application number: PCT/EP2005/001265
(87) International publication number: WO 2005/075575

(56) References cited:
- EP-A- 0 832 569
- WO-A-01/83437
- WO-A-02/12183
- WO-A-96/19215
- DE-A1- 19 841 930
- US-A- 5 310 554
- US-A- 5 612 485
- US-A1- 2002 107 292
- DATABASE WPI Section Ch, Week 199706 Derwent Publications Ltd., London, GB; Class B05, AN 1997-053021 XP002285363 & CN 1 077 190 A (UNIV NANJING) 13 October 1993 (1993-10-13)
- DATABASE INSPEC [Online] THE INSTITUTION OF ELECTRICAL ENGINEERS, STEVENAGE, GB; 15 July 1989 (1989-07-15), BAGRYANSKAYA ET. AL.: "The study of microwave-induced nuclear polarization in the sensitized trans-cis isomerization of fumaronitrile" XP002286706 Database accession no. 3454258 & BAGRYANSKAYA E G ; AVDIEVICH N I ; GRISHIN YU A ; SAGDEEV R Z: "dito" CHEMICAL PHYSICS, vol. 135, no. 1, 15 July 1989 (1989-07-15), pages 123-129, NETHERLANDS
- W. Von Doering ET AL: "Thermal interconversions among 15-cis-, 13-cis-, and all-trans-.beta.-carotene: kinetics, Arrhenius parameters, thermochemistry, and potential relevance to anticarcinogenicity of all-trans-.beta.-carotene", Journal of the American Chemical Society, vol. 117, no. 10, 1 March 1995 (1995-03-01), pages 2747-2757, XP055023033, ISSN: 0002-7863, DOI: 10.1021/ja00115a010
- WILHELM STAHL AND HELMUT SIES: "Human and Clinical Nutrition: Uptake of Lycopene and Its Geometrical Isomers Is Greater from Heat-Processed than from Unprocessed Tomato Juice in Humans", THE JOURNAL OF NUTRITION, WISTAR INSTITUTE OF ANATOMY AND BIOLOGY, vol. 122, no. 11, 1 November 1992 (1992-11-01), pages 2161-2166, XP009157953, ISSN: 0022-3166

## Description

The present invention relates to a process for the preparation of a primary composition that includes at least one carotenoid-containing material enriched in cis-isomers of the carotenoid compound.

### Background of the invention

Compositions available on the market that include carotenoids, namely lycopene, are already known. Lycopene is a natural product which is known to have multiple roles, in particular that of an antioxidant that allievates chronic diseases.

Lycopene is present in various natural products, in particular tomatoes, melons, guavas and grapefruit. The composition generally available on the market which comprises lycopene is an oleoresin. The problem with this oleoresin is that the lycopene present therein is insufficiently bioavailable.

For example, European patent 278 284 relates to a pulverulent composition comprising a synthetic carotenoid. The problem with this composition is that it cannot be used in the food field and, moreover, it is envisaged for a coloring purpose.

Although EP 1 289 383 provides a primary composition that includes at least one lipophilic bioactive compound and a whey protein in an amount effective to increase the bioavailability of the lipophilic bioactive compound, there is still a need for a carotenoid-containing product which has higher bioavailability and bioefficacy than the products currently on the market. The carotenoid-containing composition must be soluble in lipids and organic solvents" less prone to crystallization, and have a lower tendency to aggregate.

Article "Thermal interconversion among 15-cis-, 13-cis- and all trans-.beta.-carotene: kinetics, Arrhenius parameters, thermochemistry, and potential relevance to anticarcinogenicity of all-trans-.beta.-carotene", W. Von Doering et Al, Journal of the American Chemical Society, vol. 117, no. 10, 1 March 1995 (1995-03-01), pages 2747-2757), XP055023033 discloses thermal interconversion of lycopene between trans to cis lycopene in a tomato juice and the results on its absorption by the humans. The tomato juice composition once heated presents a cis:trans ratio of lycopene of 20 - 30 (all cis) : 80 - 70 (trans).

### Summary of the invention

Accordingly, it is a first object of the invention to provide a process for the preparation of a primary composition which comprises subjecting a carotenoid-containing material to conditions sufficient to increase its content in cis-isomers to an amount effective to increase the bioavailability and/or bioefficacy of the carotenoid compound in the primary composition. Said conditions are the object of claim 1.

The primary composition of the invention is further subjected to a solubilisation of *cis-*isomers in selected organic solvents followed by phase separation using centrifugation or filtration.

Advantageously and as described in the below description, the cis:trans isomer ratio of the carotenoid compound in the primary composition is from 30:70 to 90:10.

Preferably and according to the invention, the carotenoid compound is lycopene, zeaxanthine, astaxanthine, β-cryptoxanthin, capsanthine, canthaxanthine, lutein, phytofluene or phytoene, and wherein the carotenoid-containing material is in the form of an extract, a concentrate or an oleoresin.

The carotenoid-containing material is an extract, concentrate or oleoresin advantageously obtained, extracted, enriched or purified from a plant or a vegetable material, microorganism, yeast or product of animal origin.

The preferred form of the primary composition is as an additive in a foodstuff for oral administration, such as in a nutritional composition, a food supplement, a pet food product, a cosmetic preparation or in a pharmaceutical preparation.

According to the invention the primary composition is integrated in foodstuff, food supplement, cosmetic preparation or pharmaceutical preparation containing the same.

The primary composition using the process of the invention comprises at least one carotenoid-containing material enriched in cis-isomers, for the preparation of an oral, cosmetic or pharmaceutical composition intended for improving skin health, in particular for photoprotection of the skin or for protecting skin tissue against ageing.

The primary composition can be used for the preparation of an oral, cosmetic or pharmaceutical composition for preventing or treating cardiovascular diseases or cancers.

The present invention now makes available to the consumer an improved composition obtained from natural products. It provides a primary composition having a cis-isomer content of carotenoid higher than existing compositions. The primary composition provides carotenoids in a particularly highly bioavailable and/or bioeffective form, which is better soluble in lipids, less prone to crystallization, and having a lower tendency to aggregate.

The features of the present invention can be best understood together with further objects and advantages by reference to the following description, taken in connection with the accompanying drawings.

### Brief description of the drawings

Fig. 1 shows a typical HPLC chromatogram of lycopene isomers generated by microwave irradiation, illustrating the products obtained by the process according to the present invention.
Fig. 2 outlines the advantage of microwave irradiation versus conventional heating.
Fig. 3 shows comparative HPLC chromatograms of lycopene isomers: (A) generated by microwave irradiation of tomato oleoresine; (B) generated by microwave irradiation and enriched by solvent fractionation.
Fig. 4 shows HPLC chromatograms of lycopene isomers: (A) generated by iodine catalysed photoisomerization of tomato oleoresine; (B) generated by iodine catalysed photoisomerization and enriched by solvent fractionation.
Fig. 5 shows comparative HPLC chromatograms of lycopene isomers: (A) generated by heating of tomato oleoresine in ethyl acetate; (B) generated by heating of tomato oleoresine in ethyl acetate and enriched by solvent fractionation.

### Detailed description of the invention

According to the first object, a process to prepare a primary composition comprising a carotenoid-containing material enriched in cis-isomers of said carotenoid compound, is concerned.

In a preferred embodiment, the carotenoid-containing material is in the form of an extract, a concentrate or an oleoresin, for example. Within the following description, the term "oleoresin" is understood to mean a lipid extract of a carotenoid-containing material, which includes carotenoids, triglycerides, phospholipids, tocopherols, tocotrienols, phytosterols and other less significant compounds.

Advantageously , the carotenoid-containing material is an extract, concentrate or oleoresin, which is obtained, extracted, enriched or purified from a plant or vegetable material, a microorganism, a yeast or a product of animal origin. It is further subjected to a treatment to increase its cis-isomer content of carotenoid, as described below.

If the source of carotenoid is from plant origin, it may be vegetables, leaves, flowers, fruits and other parts of the plant. In a preferred embodiment, the source of carotenoids is from tomatoes (i.e., whole tomato, tomato extract, tomato flesh, tomato puree, tomato skin, with or without the seeds), carrots, peaches, apricots, oranges, melons, guavas, papayas, grapefruit, rosehips, soya, green tea, green coffee beans, spices such as ginger or others, grapes and cocoa, for example. Suitable plant or vegetable concentrates are obtainable e.g. by drying or freeze-drying the fresh-cut plants or vegetables or the respective roots, fruits or seeds thereof and then optionally grinding or granulating the dried material. Suitable methods of obtaining extracts of the above-mentioned plants or vegetables are known in the art. The plant or vegetable extracts are obtainable e.g. by extracting the fresh-cut or prosessed plants or vegetables or the respective roots, fruits or seeds thereof for example with water or with one or more food grade solvents or with a mixture of water and one or more food grade solvents. Preferably, the extrats and concentrates according to the present invention may be lipidic or aqueous. Since carotenoids are liposoluble, extraction with water will remove unwanted constituents which are water-soluble such as sugars, amino acids, soluble proteins, organic acids, for example.

If the carotenoid-containing material is obtained from microorganism, any microorganism that produces carotenoid may be used, in particular probiotic microorganism, such as lactic acid bacterium, for example. Also, the product of animal origin may be from salmon, shrimps, for example or a liver extract or a milk fraction. The term "milk fraction" is understood to mean any part of the milk.

In a most preferred embodiment, the carotenoid-containing material is an oleoresin. Suitable methods of obtaining oleoresins from the above-mentioned plants or vegetables are well known in the art. For example, oleoresin are obtained by lipidic extraction using a solvent compatible with the food business, cosmetics or pharmaceuticals. Oleoresins prepared by conventional methods have a content in carotenoid of about 0.05% to 50% by weight. Their content of all-trans isomer of carotenoids is usually higher than that of cis isomers, e.g. the ratio of cis-trans isomers of lycopene in a selected tomato oleoresin is about 7:93. Oleoresins are preferred starting material for obtaining the primary composition according to the present invention, because they contain other carotenoids or antioxidants such as Vitamine E, which also stabilize the composition. The activity and stability of the carotenoid compound in the oleoresin is improved , in particular during the isomerisation process and the yield of the cis-lycopene in the primary composition is also increased.

The carotenoid-containing material preferably includes carotenes and xanthophylls, such as lycopene, carotene, zeaxanthine, astaxanthine, beta-cryptoxanthin, capsanthine, canthaxanthine, lutein, phytofluene or phytoene, for example. Said carotenoid compounds have been subjected to a treatment to increase the cis-isomer fraction in the primary composition.

The object of the invention is to provide a process for the preparation of such a primary composition comprising the step of subjecting a carotenoid-containing material as described above, under conditions sufficient to increase its content in cis-isomers of carotenoid to an amount effective to increase the bioavailability and/or bioefficacy of the carotenoid compound in the primary composition.

The carotenoid-containing material which is in the form of an extract, a concentrate or an oleoresin, is subjected to a microwave irradiation. Conditions of the microwave irradiation depend on the quantity and quality of the material. If an oleoresin is used, the power and time are adjusted so that the temperature of the microwave oven is of at least 100°C, preferably from 100 to 180°C and most preferably from 115 to 140°C. If an aqueous extract is used, a medium adapted to microwave irradiation may be used. The aim of the medium is to solubilize or disperse carotenoids. The losses can be minimised when the isomerisation is performed under nitrogen in the presence of antioxidants and in the absence of light. The isomerisation yield may also be improved by adding exogenous lipids in the medium.

The isomers of carotenoid-containing compound generated thereof may be subjected to a further treatment intended to modify the isomer profile of the primary composition according to the intended use. The enrichment in some specific *cis-*isomers may be achieved by solubilisation of cis-isomers in selected organic solvents followed by phase separation using centrifugation or filtration, for example.

The *cis:trans* isomer ratio in the primary composition may then be increased up to at least 20:80, preferably between 20:80 and 95:5, more preferably from from 30:70 to 90:10.

The process of the present invention thus leads to a primary composition, in the form of a powder, liquid or gel, comprising a carotenoid compound which has a better bioavailability and/or bioefficacy than the compound alone. Also, the primary composition may be in the form of a highly water-dispersible composition, if the powder form is chosen. In this instance, the powder is dispersible in water at ambient temperature. The primary composition also provides carotenoids in a particularly highly soluble form in lipids and organic solvents, less prone to crystallization, and having a lower tendency to aggregate.

The primary composition obtained by the inventive process may be used either alone or in association with other active compounds such as vitamin C, vitamin E (tocopherols and tocotrienols), carotenoids (carotenes, lycopene, lutein, zeaxanthine, beta-cryptoxanthine, etc ..) ubiquinones (e.g. CoQ₁₀), catechins (e.g. epigalloc atechin gallate), coffee extracts containing polyphenols and/or diterpenes (e.g. kawheol and cafestol), extracts of chicory, ginkgo biloba extracts, grape or grape seed extracts rich in proanthocyanidins, spice extracts (e.g. rosemary), soy extracts containing isoflavones and related phytoestrogens and other sources of flavonoids with antioxidant activity, fatty acids, e.g. n-3 fatty acids, phytosterols, prebiotic fibers, probiotic microorganisms, taurine, resveratrol, aminoacids, selenium and precursors of gluthathione, or proteins, such as whey proteins, for example.

The composition additionally comprises one or more of emulsifiers, stabilizers and other additives. Use is made of emulsifiers compatible in the food field, such as phospholipids, for example lecithin; polyoxyethylene sorbitan mono- or tris-tearate, monolaurate, monopalmitate, mono- or trioleate; a mono- or diglyceride. Use may also be made of any type of stabilizer that is known in the food business, in cosmetics or in pharmaceuticals. Use is made, as additives, of flavorings, colorants and any other additive known in the food business, in cosmetics or in pharmaceuticals. These emulsifiers, stabilizers and additives are added according to the final use of the primary composition.

A further embodiment relates to an oral composition comprising the primary composition described above in a foodstuff, in a food supplement, in a pet food product, in a cosmetic preparation or in a pharmaceutical preparation.

In a further embodiment, a food composition for human consumption is supplemented by the above primary composition. This composition may be a nutritional complete formula, a dairy product, a chilled or shelf stable beverage, a mineral water, a liquid drink, a soup, a dietary supplement, a meal replacement, a nutritional bar, a confectionery, a milk or a fermented milk product, a yogurt, a milk based powder, an enteral nutrition product, an infant formulae, an infant nutritional product, a cereal product or a fermented cereal based product, an ice-cream, a chocolate, coffee, a culinary product such as mayonnaise, tomato puree or salad dressings or a pet food.

In this case, the powder is dissolved in the above-mentioned foods or drinks so as to have a daily intake of between about 0.001 and 50 mg of carotenoid contained in the primary composition, for example such as lycopene. A daily intake of the order of about 5 to 20 mg per day is preferably envisaged.

The nutritional supplement for oral administration may be in capsules, gelatin capsules, soft capsules, tablets, sugar-coated tablets, pills, pastes or pastilles, gums, or drinkable solutions or emulsions, syrups or gels, with a dose of about 0.001 to 100% of the primary composition, which can then be taken directly with water or by any other known means. This supplement may also include a sweetener, a stabilizer, an additive, a flavoring or a colorant. A supplement for cosmetic purpose can additionally comprises a compound active with respect to the skin. Methods for preparing them are common knowledge.

In another embodiment, a pharmaceutical composition can be administered for prophylactic and/or therapeutic treatments. In therapeutic applications, compositions are administered to a patient already suffering from a disease, as described herein under, in an amount sufficient to cure or at least partially arrest the symptoms of the disease and its complications. An amount adequate to accomplish this is defined as "a therapeutically effective dose". Amounts effective for this will depend on the severity of the disease and the weight and general state of the patient.

In prophylactic applications, compositions according to the embodiments are administered to a patient susceptible to or otherwise at risk of a particular disease. Such an amount is defined to be "a prophylactic effective dose". In this use, the precise amounts again depend on the patient's state of health and weight.

The compounds of the herein described embodiments are preferably administered with a pharmaceutical acceptable carrier, the nature of the carrier differing with the mode of administration, for example parenteral, intravenous, oral and topical (including ophthalmic) routes. The desired formulation can be made using a variety of excipients including , for example, pharmaceutical grades of mannitol, lactose, starch, magnesium stearate, sodium saccharin cellulose, magnesium carbonate. This composition may be a tablet, a capsule, a pill, a solution, a suspension, a syrup, a dried oral supplement, a wet oral supplement, dry tube-feeding, wet tube-feeding etc..

Preferably, for humans the pharmaceutical composition according to the present invention comprises an amount of the primary composition as described above, for a daily administration, so that the carotenoid amount is from about 0.01 mg to 100 mg. When administered daily to pets, the carotenoid amount is from about 0.01 mg to 100 mg.

It will be appreciated that the skilled person will, based on his own knowledge select the appropriate components and galenic form to target the active compound to the tissue of interest, e.g. the skin, colon, stomach, kidney or liver, taking into account the route of administration which may be by way of injection, topical application, intranasal administration, administration by implanted or transdermal sustained release systems, and the like.

The process of the present invention also relates to a cosmetic composition comprising the primary composition described above. It may be formulated in lotions, shampoos, creams, sunscreens, after-sun creams, anti-ageing creams and/or ointments, for example. In this case, the content of primary composition is between 10.sup.-10 and 10%. The cosmetic composition preferably comprises between 10.sup.-8 and 5% of carotenoid compound. This composition which can be used topically additionally comprises a fat or an oil which can be used in cosmetics, for example those mentioned in the CTFA work, Cosmetic Ingredients Handbook, Washington. It is also possible to add other cosmetically active ingredients. The composition additionally comprises a structuring agent and an emulsifier. Other excipients, colorants, fragrances or opacifiers can also be added to the composition. It will be appreciated that the present cosmetic products will contain a mixture of different ingredients known to the skilled person, ensuring a fast penetration of the objective substance into the skin and preventing degradation thereof during storage.

The effect of a food supplementation in the primary composition according to the process of the present invention, on skin of humans or pets, can be measured by using conventional methods including minimal erythemal dose (MED), colorimetry, transepidermal water loss, DNA repair (e.g. p.53), measure of interleukines and proteoglycans production, or collagenase activity, barrier function or cell renewal or ultrasonic echography.

The following examples illustrate the invention in more detail without restricting the same thereto. All percentages are given by weight otherwise indicated.

### Examples

### Example 1: Preparation of a primary composition by microwave treatment

25 g of lycopen oleoresin manufactured by LycoRed Natural Products Industries, Ltd. in Israel, are subjected to a microwave treatment of 150 sec. at 2.45 GHz at a temperature of about 105°C. The primary composition thus obtained is cooled down under nitrogen to prevent carotenoid degradation. The ratio of *cis:trans* isomer for lycopene is about 65:35 in the resulting composition.

### Example 2 (Reference):

### Preparation of a primary composition by thermal treatment

0.28 g of oleoresin (Indena 10% lycopene) in 30 mL of ethyl acetate is isomerised by heating for 1h under reflux in a nitrogen atmosphere and evaporation of the solvent under reduced pressure. The *cis:trans* isomer ratio is about 35% after heating

### Example 3 (Reference):

### Preparation of a primary composition by radical reaction

2 g oleoresin (Indena 10% lycopene) in 100 mL CH₂Cl₂ are mixed with 116 µL Iodine solution (2.8 mg Iodine in 298 µL CH₂Cl₂). The mixture is photoisomerised for 1h 50 at room temperature and the solvent removed under reduced pressure. The *cis:trans* isomer ratio is about 77% after photoisomerisation.

### Example 4 (Reference):

### Preparation of a primary composition by thermal reaction

10 g of tomato paste (Thomy) are mixed with 10 g of high oleic sunflower oil (Trisun) at room temperature. The mixture is heated using a plate heater for 15 minutes (final temperature 100 °C). The *cis:trans* isomer ratio equals to 40 % after heating.

### Example 5: Comparison of lycopene isomerisation by microvawe treatment and conventional heating

The isomerisation yield of a tomato oleoresin from LycoRed Natural Products Industries, Ltd. in Israel, which has been treated by microwave or conventional heating (oil bath) is measured.

For this, tomato oleoresin (Lycored 6%) in glass vials was irradiated in a microwave oven (2.45 GHz) for various periods of time up to 150 s. At the end of each exposure period the temperature was measured in the oleoresin sample. After cooling, the samples were dissolved in n-hexane (containing 200 ppm BHT) and analysed by HPLC for isomer formation. Additionally, tomato oleoresins in glass vials were heated in an oil bath to the same temperatures as those previously measured at the end of the microwave treatments. They were removed immediatedly from the oil bath, left for cooling and analysed in the same way as the microwave irradiated samples. The HPLC method for lycopene isomer determination was adapted from Schierle et al. (1997) Food Chem. 59,459-65.

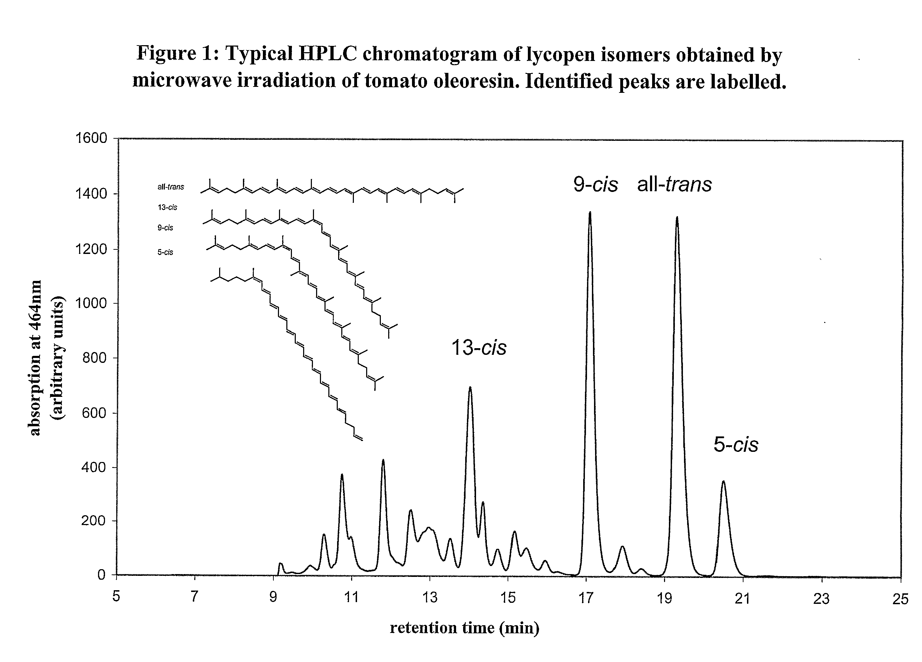

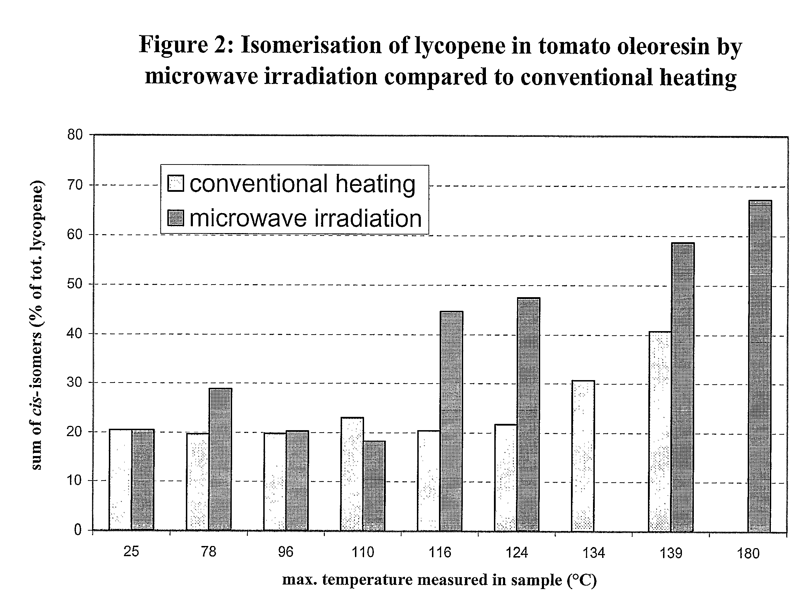

The results are illustrated in Figs 1 and 2. Fig. 1 shows that predominantly 9-and *13-cis* isomers of lycopene were generated by microwave irradiation, beside some other unidentified isomers and remaining *all-trans* lycopene. Fig. 2 shows that lycopene isomerisation is stronger in the microwave treated oleoresin and the temperatures needed to generate *cis* isomers were lower compared to the conventionally heated samples.

In conclusion microwave is a very efficient method to generate geometrical isomers of lycopene.

### Example 6: Amount of lycopene cis-isomers in processed and fractionated tomato oleoresin

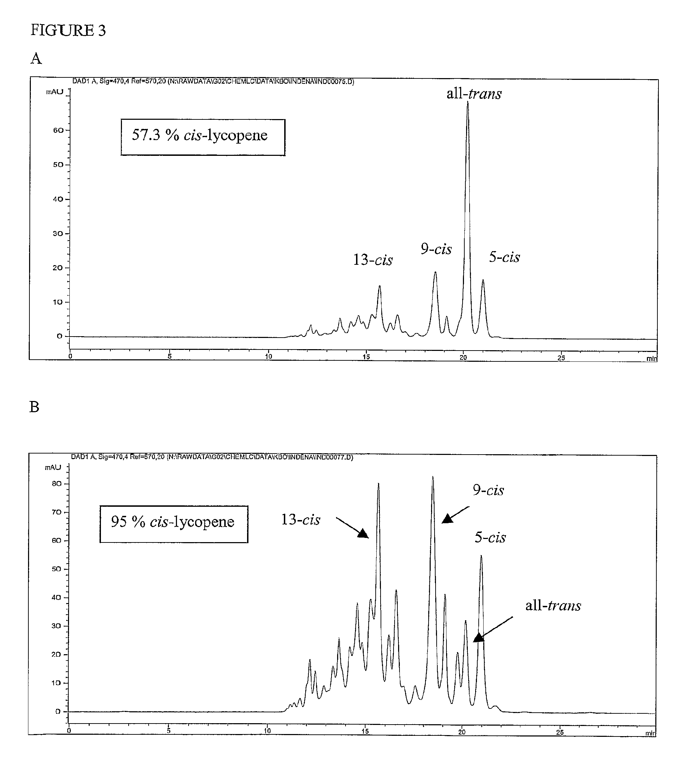

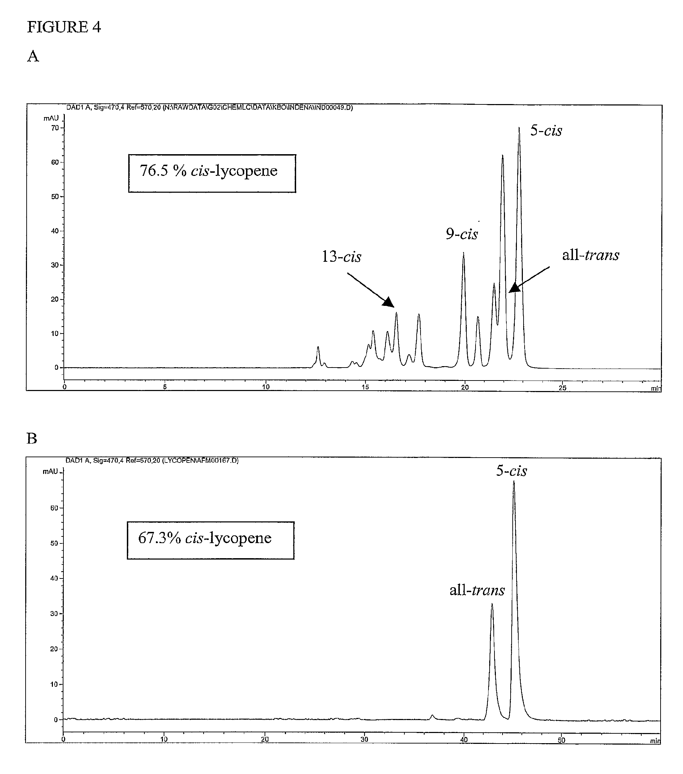

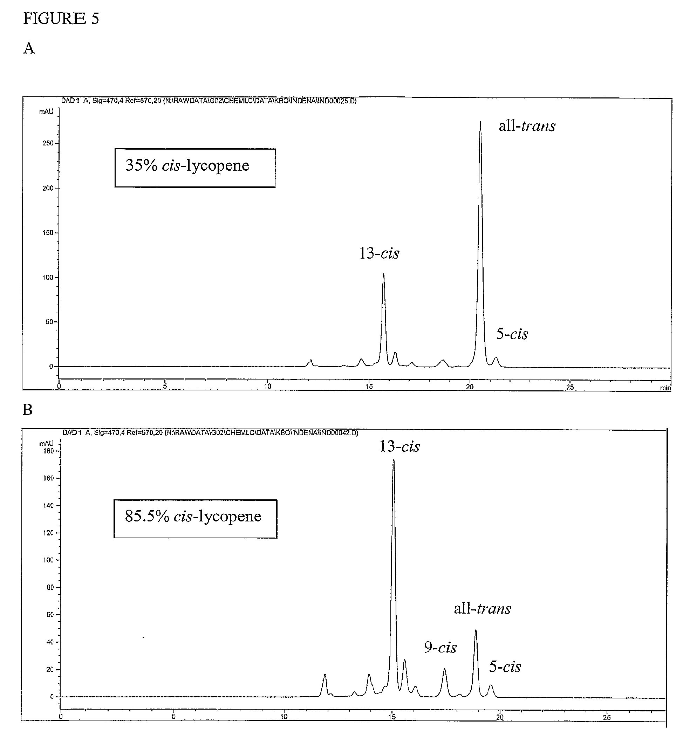

The profile of lycopene isomers in a tomato oleoresin from Indena, spa., is determined by HPLC as in Example 5, to compare the following treatments :
- isomerisation of lycopene by microwave treatment of 2 g oleoresin (Indena, 10%) at 2.45 GHz for 90 s (fig. 3A) followed by resuspension in ethanol, centrifugation (13'000 x g, 5 min, ambient temperature), rejection of the solid fraction and evaporation of the solvent under reduced pressure (fig. 3B)
- generation of lycopene isomers by iodine catalysed photoisomerisation (1h 50) of 2 g oleoresin (Indena 10%) in CH₂Cl₂ (fig 4A), followed by evaporation of CH₂Cl₂, resuspension in ethanol, centrifugation (13'000 x g, 5 min, ambient temperature), resuspension of the solid fraction in ethyl acetate, centrifugation (13'000 x g, 5 min, ambient temperature), rejection of the solid fraction and evaporation of the solvent under reduced pressure (fig. 4B)
- isomerisation of lycopene by refluxing 0.28 g of oleoresin (Indena 10%) in 30 mL of ethyl acetate for 1h (fig 5A), followed by evaporation of ethyl acetate, resuspension in ethanol, centrifugation (13'000 x g, 5 min, ambient temperature), rejection of the solid fraction and evaporation of the solvent under reduced pressure (fig 5B)

### Results

Fig 3A shows that MW treatment of tomato oleoresin induced the formation of a broad range of lycopene cis-isomers. Fig 3B shows that solvent fractionation specifically increases *cis* to *trans* isomer ratio in MW treated tomato oleoresin.

Fig. 4A shows that predominantly the *5-cis* isomer is formed during photoisomerisation of tomato oleoresin. Fig. 4B shows that almost exclusively the all-*trans* and the *5-cis* isomers are recovered after solvent fractionation.

Fig 5A shows that the *13-cis* isomer is predominantly formed during short time refluxing tomato oleoresin in ethyl acetate. Fig. 5B shows that mainly the *13-cis* isomer is retained in the soluble fraction after solvent fractionation.

### Example 7: Cosmetic for oral administration

A composition in the form of a hard capsule has the following formulation:

| Compound | mg per capsule |
|---|---|
| primary composition of example 1 | 250 |
| | |

| **Excipient for the coating core** | |
|---|---|
| Microcrystalline cellulose | 70 |
| Encompress TM | 60 |
| Magnesium stearate | 3 |
| Anhydrous colloidal silica | 1 |

| **Coating agent** | |
|---|---|
| Gum-lac | 5 |
| Talc | 61 |
| Sucrose | 250 |
| Polyvidone | 6 |
| Titanium dioxide | 0.3 |
| Coloring agent | 5 |

The composition can be administered to the individual in an amount of 2 to 3 capsules daily.

## Claims

1. A process for the preparation of a primary composition, which comprises subjecting a carotenoid-containing material to a treatment increasing its content in cis-isomers of the carotenoid compound in which said treatment, is microwave irradiation.

2. The process according to claim 1, in which the primary composition is further subjected to a solubilisation of cis-isomers in selected organic solvents followed by phase separation using centrifugation or filtration.

3. The process according to any of claims 1 or 2 wherein the cis:trans isomer ratio of the carotenoid compound in the primary composition is from 30:70 to 90:10.

4. The process according to any of claims 1 to 3 wherein the carotenoid compound is lycopene, zeaxanthine, astaxanthine, β-cryptoxanthin, capsanthine, canthaxanthine, lutein, phytofluene or phytoene, and wherein the carotenoid-containing material is in the form of an extract, a concentrate or an oleoresin.

5. The process according to any of claims 1 to 4 wherein the carotenoid containing material is obtained, extracted or purified from a plant or vegetable material, microorganism, yeast or product of animal origin.

6. The process according to claim 5, wherein the plant or vegetable material is tomatoes, carrots, peaches, apricots, oranges, melons, guavas, papayas, grapefruit, rosehips, soya, green tea, green coffee beans, spices, grapes or cocoa.

7. The process according to any of claims 1 to 6, wherein the primary composition is in liquid, gel or powder form.

8. The process according to any of claims 1 to 7 wherein the primary composition is integrated in a foodstuff, in a food supplement, in a pet food product, in a cosmetic preparation or in a pharmaceutical preparation.

9. The process according to claim 8, wherein the foodstuff is chosen from the group consisting of a nutritional complete formula, a dairy product, a chilled or shelf stable beverage, a mineral water, a liquid drink, a soup, a dietary supplement, a meal replacement, a nutritional bar, a confectionery product, a milk or a fermented milk product, a yogurt, a milk based powder, an enteral nutrition product, an infant formulae, an infant nutritional product, a cereal product or a fermented cereal based product, an ice-cream, a chocolate, coffee, a culinary product or a pet food product.

## Patentansprüche

1. Verfahren zur Herstellung einer Primärzusammensetzung, welches das Unterziehen eines Carotenoid-enthaltenden Materials einer Behandlung, die den Gehalt an cis-Isomeren der Carotenoidverbindung erhöht, wobei die Behandlung eine Mikrowellenbestrahlung ist.

2. Verfahren nach Anspruch 1, wobei die Primärzusammensetzung ferner einer Solubilisierung von cis-Isomeren in ausgewählten organischen Lösungsmitteln unterzogen wird, gefolgt von Phasentrennung unter Verwendung von Zentrifugierung oder Filtrierung.

3. Verfahren nach einem der Ansprüche 1 oder 2, wobei das cis:trans-Isomerverhältnis der Carotenoidverbindung in der Primärzusammensetzung von 30:70 bis 90:10 beträgt.

4. Verfahren nach einem der Ansprüche 1 bis 3, wobei die Carotenoidverbindung Lycopen, Zeaxanthin, Astaxanthin, β-Cryptoxanthin, Capsanthin, Canthaxanthin, Lutein, Phytofluen oder Phytoen ist, und wobei das Carotenoid-enthaltende Material in der Form eines Extraktes, eines Konzentrats oder eines Oleoresins vorliegt.

5. Verfahren nach einem der Ansprüche 1 bis 4, wobei das Carotenoid-enthaltende Material aus einem Pflanzen- oder Gemüsematerial, Mikroorganismus, Hefe oder einem Produkt tierischen Ursprungs erhalten, extrahiert oder gereinigt wird.

6. Verfahren nach Anspruch 5, wobei das Pflanzen- oder Gemüsematerial Tomaten, Karotten, Pfirsiche, Aprikosen, Orangen, Melonen, Guaven, Papayas, Grapefruit, Hagebutten, Soja, grüner Tee, grüne Kaffeebohnen, Gewürze, Trauben oder Kakao ist.

7. Verfahren nach einem der Ansprüche 1 bis 6, wobei die Primärzusammensetzung in flüssiger Form, Gelform oder Pulverform vorliegt.

8. Verfahren nach einem der Ansprüche 1 bis 7, wobei die Primärzusammensetzung in einem Nahrungsmittel, einer Nahrungsergänzung, einem Haustiernahrungsprodukt, einer kosmetischen Zusammensetzung oder einer pharmazeutischen Zusammensetzung integriert ist.

9. Verfahren nach Anspruch 8, wobei das Nahrungsmittel ausgewählt ist aus der Gruppe bestehend aus einer Vollnahrungsformel, einem Milchprodukt, einem gekühlten oder haltbaren Getränk, einem Mineralwasser, einem flüssigen Getränk, einer Suppe, einem Nahrungsergänzungsmittel, einem Mahlzeitenersatz, einem Ernährungsriegel, einem Süßwarenprodukt, einem Milch- oder fermentierten Milchprodukt, einem Joghurt, einem auf Milch basierenden Pulver, einem enteralen Ernährungsprodukt, einer Säuglingsnahrung, einem Säuglingsernährungsprodukt, einem Getreideprodukt oder einem auf fermentiertem Getreide basierenden Produkt, einem Speiseeis, einer Schokolade, Kaffee, einem kulinarischen Produkt oder einem Haustiernahrungsprodukt.

## Revendications

1. Procédé de préparation d'une composition primaire, qui comprend la soumission d'un matériau contenant un caroténoïde à un traitement augmentant sa teneur en isomères *cis* du composé caroténoïde dans lequel ledit traitement est une irradiation par micro-ondes.

2. Procédé selon la revendication 1, dans lequel la composition primaire est en outre soumise à une solubilisation des isomères *cis* dans des solvants organiques sélectionnés, suivie par une séparation des phases à l'aide d'une centrifugation ou d'une filtration.

3. Procédé selon l'une quelconque des revendications 1 ou 2, dans lequel le rapport isomérique cis:trans du composé caroténoïde dans la composition primaire va de 30:70 à 90:10.

4. Procédé selon l'une quelconque des revendications 1 à 3, dans lequel le composé caroténoïde est le lycopène, la zéaxanthine, l'astaxanthine, la β-cryptoxanthine, la capsanthine, la canthaxanthine, la lutéine, le phytofluène ou le phytoène, et dans lequel le matériau contenant un caroténoïde se présente sous la forme d'un extrait, d'un concentré ou d'une oléorésine.

5. Procédé selon l'une quelconque des revendications 1 à 4, dans lequel le matériau contenant un caroténoïde est obtenu, extrait ou purifié à partir d'une plante ou d'un matériau végétal, d'un micro-organisme, d'une levure ou d'un produit d'origine animale.

6. Procédé selon la revendication 5, dans lequel la plante ou le matériau végétal est des tomates, des carottes, des pêches, des abricots, des oranges, des melons, des goyaves, des papayes, du pamplemousse, des cynorhodons, du soja, du thé vert, des fèves de café vertes, des épices, des raisins ou du cacao.

7. Procédé selon l'une quelconque des revendications 1 à 6, dans lequel la composition primaire est sous forme de liquide, de gel ou de poudre.

8. Procédé selon l'une quelconque des revendications 1 à 7, dans lequel la composition primaire est intégrée dans un produit alimentaire, dans un complément alimentaire, dans un produit alimentaire pour animal de compagnie, dans une préparation cosmétique ou dans une préparation pharmaceutique.

9. Procédé selon la revendication 8, dans lequel le produit alimentaire est choisi parmi le groupe constitué d'une préparation nutritionnelle complète, un produit laitier, une boisson glacée ou à longue durée de conservation, une eau minérale, une boisson liquide, une soupe, un complément diététique, un substitut de repas, une barre nutritionnelle, un produit de confiserie, un lait ou un produit de lait fermenté, un yaourt, une poudre à base de lait, un produit de nutrition entérale, une préparation pour nourrissons, un produit nutritionnel pour nourrissons, un produit de céréales ou un produit fermenté à base de céréales, une crème glacée, un chocolat, un café, un produit culinaire ou un produit alimentaire pour animal de compagnie.
